# EUROPEAN PATENT SPECIFICATION

(11) **EP 1 529 847 B1**
(45) Date of publication and mention of the grant of the patent: **05.04.2006**
(21) Application number: 03090379.3
(22) Date of filing: 07.11.2003
(51) Int. Cl.: C12Q 1/68

(54) **Method for detecting methicillin resistant Staphylococcus aureus (MRSA)**
Verfahren zur identifizierung von Methicilin resistente Stapylococcus aureus (MRSA)
Méthode pour la détection de Staphylococcus aureus résistant aux méthiciline (MRSA)

(43) Date of publication of application: 11.05.2005
(73) Proprietor: Federal Rep. of Germany repr. by the Ministry of Health & Soc. Security, the latter repr. by the Pres. of the Robert Koch Ins., 13353 Berlin (DE)
(72) Inventor: Cuny, Christa, D-38855 Silstedt (DE); Witte Wolfgang Prof.Dr., D-38875 Elend (DE)
(74) Representative: Bittner, Thomas L.

(56) References cited:
- EP-A- 1 160 333
- WO-A-02/099034
- WO-A-03/039703
- DATABASE EMBL [Online] "Sequence 98 from patent WO2099034" retrieved from EBI Database accession no. AX720521.1 XP002274914
- DATABASE EMBL [Online] 22 February 2001 (2001-02-22) KEIICHI, AWAYA: "Identification Method" retrieved from EBI Database accession no. E60315 XP002274915 & EP 0 887 424 A (KAINOS LAB INC) 30 December 1998 (1998-12-30)
- HULETSKY A ET AL: "DEVELOPMENT OF A REAL-TIME PCR ASSAY FOR RAPID DETECTION OF METHICILLIN-RESISTANT STAPHYLOCOCCUS AUREUS DIRECTLY FROM CLINICAL SPECIMENS CONTAINING A MIXTURE OF STAPHYLOCOCCI" ABSTRACTS OF THE GENERAL MEETING OF THE AMERICAN SOCIETY FOR MICROBIOLOGY, THE SOCIETY, WASHINGTON, DC, US, vol. 102, 19 May 2002 (2002-05-19), page 143 XP009025123 ISSN: 1060-2011

## Description

### Background of the invention

Since its first identification in the early 1960s (Jevons, M. P. 1961 "Celbenin" resistant staphylococci), methicillin-resistant *Staphylococcus aureus* (MRSA) has become one of the most significant nosocmial pathogens throughout the world and it is capable of causing a wide range of hospital infections. It continues to spread through new communities wherever the methods and institutions of modem medical practice are adopted, while it regularly causes epidemics in places where it has been endemic for a decade or more. Consequently, the analysis and of the dissemination of MRSA isolates has been a research focus for a decade or more.

### Drug Resistance and Clinical Features associated with Staphylococcus aureus

*Staphylococcus aureus* has long been recognised as one of the major human pathogens responsible for a wide range of afflictions from minor infections of the skin to wound infections, bacteraemia, infections of the central nervous system, respiratory and urinary tracts, and infections associated with intravascular devices and foreign bodies. *Staphylococcus aureus* infections can be lethal. Most *Staphylococcus aureus* strains are opportunistic pathogens that can colonise individuals, without symptoms, for either short or extended periods of time, causing disease when the immune system becomes compromised. The immense genetic repertoire of this bacterium for adapting to rapidly changing and uniformly hostile environments was repeatedly shown by the emergence of *Staphylococcus aureus* strains that acquired resistance mechanisms to virtually all antimicrobial agents shortly after the introduction of these drugs into clinical practice. A study has shown that 97% of the *Staphylococcus aureus* isolates recovered carried the resistant trait to penicillin (Sa-Leao R. et al, Microb. Drug. Res. 2001; 7: 237-245).

The introduction of methicillin in clinical practice in 1960 was followed by the appearance of the first blood stream isolate of *Staphylococcus aureus* that was resistant not only to penicillin, streptomycin, and tetracycline (and occasionally to erythromycin), but to methicillin as well. Since the 1960s, MRSA strains have spread among hospitals isolates in several waves, which eventually disseminated these strains world wide.

The genus can be divided into two groups: *Staphylococcus aureus* which is a pathogen of humans and the group of coagulase-negative staphylococci (CNS) which are usually part of the physiological skin flora.

### Coagulase-negative staphylococci (CNS)

As part of the physiological flora of the skin and mucous membranes of humans and animals *S. epidermidis, S. hominis, S. saprophyticus and S. haemolyticus* are usually the predominant species in humans. Whereas CNS were previously generally considered to be non-pathogenic, recent clinical experience has shown that these organisms can indeed trigger infectious processes. They are among the most frequent sepsis pathogens particularly in immunodeficient patients as well as in premature infants and neonates. S. *epidermidis* dominates in infections associated with implanted foreign bodies and intravasal catheters due to its ability to adhere irreversibly to plastic surfaces.

Nevertheless a positive test for CNS in clinical specimens must be judged critically since in most cases it is due to exogenous or endogenous contamination.

### Staphylococcus aureus

*Staphylococcus aureus* is the most potent pathogen of the genus and causes infections as well as toxin-mediated diseases. It is part of the normal skin flora in healthy persons and is mainly found in the anterior nasal sinus, in the throat, the openings of the mammary glands and on the skin (armpit, perineal region and neck). However, it can cause severe infections when the general condition of the patient is weakened and after tissue injury, surgical interventions etc. *S. aureus is* described as worldwide the most frequent cause of sepsis, skin and soft-tissue infections, and pneumonia.

Hence a diagnostic differentiation of CNS from *Staphylococcus aureus* is absolutely essential.

A number of attempts have been made to develop diagnostic procedures which are suitable for distinguishing *Staphylococcus aureus* from CNS. US patent 6,156,507 discloses a method wherein a first primer located in the chromosomal surrounding DNA (CSDNA), in this case the 5' region, and a second primer is located in the SCC*mec* integration region. The disclosed procedure has numerous drawbacks. Firstly the amplicons which are generated are extremely large in size (see below) and secondly it can be shown that the method is only applicable for a subset of MRSA types and strains. It should be noted, that a routine diagnostic method which requires the creation of amplicons larger than 1.5 kb in size is virtually useless in a clinical bacteriological laboratory due to the fact the repeatable reliable experimental amplification procedure can not be guaranteed and additionally the subsequent detection of an amplimer of said size requires specific gel electrophoresis procedures which are also not suitable for a hospital environment. The primer disclosed in US Patent no. 6,156,507 for specific detection of MRSA is primer Nmec5: 5'-ATG CTC TTT GTT TTG CAG CA at pos. 33.258 in AB033763 for SCCmec I and 56.942 in D86934 for SCCmec II resulting in an amplimer size of 5585 bp for the ORFX for SCCmec I, an amplimer size of 1259 bp for SCCmec II, an amplimer size of 682 bp. (Pos. 23.512 in AB063172) in SCCmec IVa, an amplimer size of 693 bp (pos. 20.509 in ABO63173) in SCCmec IVb and Gmec 1045:5`-ATA TTC TAG ATC ATC AAT AGT TG at pos. 10.030 in ABO4708 for SCCmec III with an amplimer size of 20.026 bp for SCCmec III. Primer J 6: 5'-GTT CAA GCC CAG AAG CGA TGT was not detectable in the above mentioned gene bank annotations. Both size distribution as well as size make the use of these primers impossible. In particular direct detection in reverse hybridization is not possible.

The inventors have demonstrated herein that in fact only 1 of 3 epidemic strains possessing SCCmec type II present in Germany were correctly analyzed (unpublished).

Additionally, as outlined in WO 02/099034 the set of primers described by Hiramatsu et al. in US patent 6,156,507 gave results showing the only twenty of the 39 MRSA strains tested in said WO 02/099034 patent application were not amplifiable. Hiramitsu's method indeed was successful in detecting less than 50% of the tested 39 MRSA strains.

The results demonstrate that some MRSA strains have sequences at the right extremity of the SCC*mec* chromosome that may not be amplified with the method disclosed in US patent 6,156,507. Consequently, the system developed by Hiramatsu et al. does not allow the detection of ali MRSA strains.

In contrast WO 02/099034 discloses a method wherein small amplicons are created. Here, the primers or probes are also located in the chromosomal surrounding DNA (CSDNA), in this case however, the 3' region, as well as in the SCC*mec* integration region. The disclosed method attempts to solve the problem of sequence variability by providing for a great number of varying probes or primers which are meant to encompass the entire sequence space of strains and/or MRSA, MSSA etc. types known to date. As can be shown in experiments performed by the inventors on 4 strains with until now not described SCCmec's herein, this solution equally leaves a great risk of not detecting particular strains and/or types. Primers 66, 67, 112, 115, and 116 used separately in combination with a reverse primer in ORFX failed to give an amplimer with 4 German MRSA containing presently unknown SCCmec cassettes. More importantly the method inherently carries the great risk of laboratory contamination due to the numerous amount of probes applied and great complexity of the amplification reaction. Additionally, novel primers have to be introduced newly according taking the evolution of MRSA into account. That means this system has to be adapted continuously to the actual epidemiological situation.

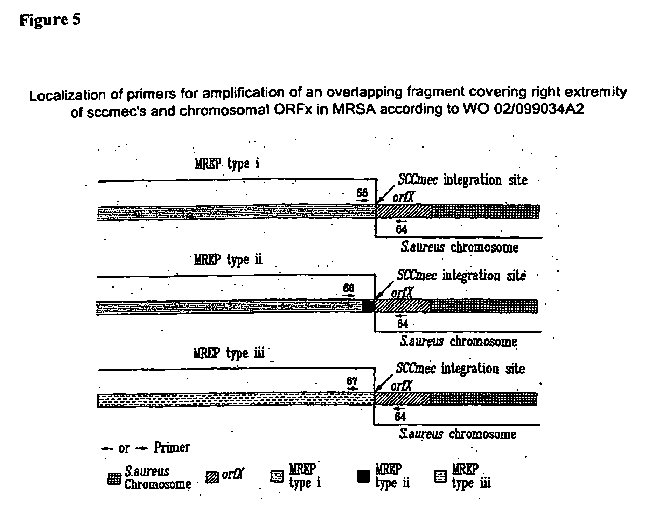

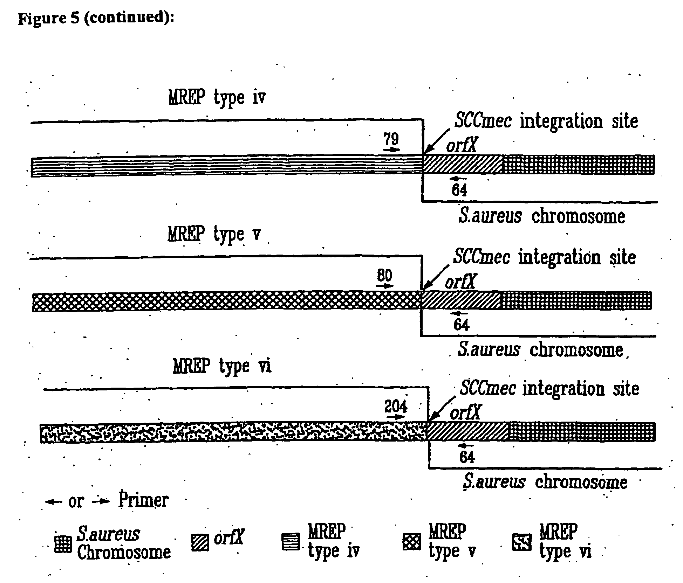

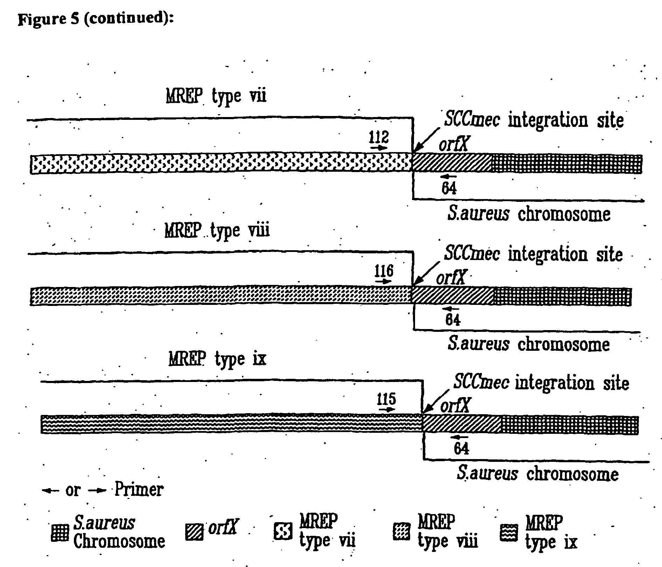

The primers used are shown in table 1, the location of the binding sites in different SCCmec-types in Figure 5.

Table 1: Primers disclosed in WO 02/099034 for amplification of an overlapping fragment of SCCmec-types and ORFX in different SCCmec-types

Attribution of primers for specific detection of SCCmec's according to patent WO 02/009034 A2 to SCCmec-sequences from accessible data banks

| SCCmec-type | Acc. no. | 66 | 67 | 112 | 116 | 115 | ORFX 1 |
|---|---|---|---|---|---|---|---|
| I | AB033763 amplimer size: 176 | +¹⁾ | - | - | - | - | +¹⁰⁾ |
| II | ***D86934*** amplimer size: 277 | +²⁾ | - | - | - | +⁷⁾ | +¹¹⁾ |
| IIIa | AB047088 | - | +⁵⁾ | - | - | - | +¹²⁾ |
| IIIb | AB047089 | - | +⁶⁾ | - | - | - | +¹³⁾ |
| IVa | (AB063172) amplimer size: 275 | +³⁾ | - | - | - | +/+⁸⁾ | +¹⁴⁾ |
| IVb | (AB063173) amplimer size: 277 | +⁴⁾ | - | - | - | +⁹⁾ | +¹⁵⁾ |

| | | | | | | | |
|---|---|---|---|---|---|---|---|
| ¹⁾ 38.830 bp →; ²⁾ 57.514 bp →; ³⁾ 25.085 bp →; ⁴⁾ 21.080 →; ⁵⁾ 67.710 bp →; 6) 23.206 bp → (location at nucleotide numbers downstream) 112 → no homology 116 → no homology ⁷⁾ 57.564 bp →; ⁸⁾ 25.133 bp → (0-1 mismatch (W)) + (0-1 1 mismatch - W) 4104 bp → (1-2 mismatches - W); ⁹) 21.130 bp → (0-1 mismatch - W) ¹⁰⁾ 39.005 bp ←; ¹¹⁾ 57.791 bp ←; ¹²⁾ 67.932 bp ←;¹³⁾23.428 bp ←;¹⁴⁾25.360 ←; (location at nucleotide numbers upstream) | | | | | | | |

The attribution of these primers to sequences of SCCmec as annotated in data banks is shown in table 2.

For covering detection of all four fully described SCCmec-types at least three different forward primers are needed (66, 67 and 115). For covering SCC-mec's in until now unknown Canadian MRSA two additional primers, namely 112 and 116 had to be introduced.

In summary, methods have been disclosed so far that have made use of primer pairs wherein either the up-stream primer or the down-stream primer was located entirely in the SCC*mec* integration region and the opposing primer was located in the CSDNA.

To date these methods have either failed do to the fact that they were not applicable to the majority of or the necessary number of clinically relevant strains and/or types, they were not suited for an application in a hospital environment.

Thus, it is the object of this invention is to provide for a reliable and rapid method for distinguishing *Staphylococcus aureus* MRSA strains of various SCC*mec* types from CNS. It is an object of this invention to provide for a method which may be applied in a hospital environment. It is an object of this invention to provide for a method which works robustly, is reliable and easy to automate or be applied to the use as a commercial diagnostic kit.

### Detailed Description of the Invention

The invention provides for a method of detecting a methicillin resistant Staphylococcus aureus strain in a sample, said strain comprising a chromosomal staphylococcal cassette chromosome mec (SCCmec) insert containing a mecA gene, wherein the Staphylococcus aureus strain may be chosen from the group comprising Staphylococcus aureus type I, type II, type III, type IV and wherein the method comprises the steps of, (i) performing a sequence specific amplification reaction wherein, the amplification re-action comprises one or more first nucleic acid primers, wherein said first nucleic acid primer has a sequence according to SEQ ID NO. 1 or SEQ ID NO. 5, the 5' end of which lies within the SCCmec region of the bacterial chromosome and 3' end of which lies in the chromosomal surrounding DNA (CSDNA), at least a second nucleic acid primer is present in the reaction which lies in the downstream CSDNA and which serves as a downstream opposing primer to said first primer, and (ii)detecting the presence or absence of an amplification product from step (i).

The prior art discloses various attempts of creating a PCR based method wherein in all cases the probes and/or primers were located both upstream and downstream either of the 5' junction between SCC*mec* and ORFX or the 3' junction between SCC*mec* and ORFX (see for example Figure 5).

In contrast this invention discloses a method wherein one or more first nucleic acid primers are situated such that the 5' end lies within the SCC*mec* region of the bacterial chromosome and 3' end of said one or more first nucleic acid primers lies in the chromosomal surrounding DNA (CSDNA) which is sometimes herein also referred to as ORFX region or ORFX DNA (see also figure 5). As can be seen in figure 16 the uniqueness of the method lies in the fact that in contrast to all rules of designing primers for PCR said first one or more nucleic acid primers are located such that the 3' end lies in a region which both CNS as well as MRSA strains contain. Ordinarily one skilled in the art would design a primer in such a way that the 3' end lies within the region which is to be detected. It is known in the art the 3'-end of primers are necessary for binding of the polymerase to the primer-template complex. One skilled in the art would not assume that the 5' end of a primer is able, under certain conditions to lead to sufficient specificity, that one would place a primer in such a way that its 3' end lies within a region that all samples comprise. For example the Amplification Refractory Mutation System (ARMS) also known as "Allele Specific PCR (ASPCR)" and the PCR Amplification of Specific Alleles (PASA) for detection of known single-base substitutions or microdelet/insertions (Sommer et al 1989 Mayo Clinic Proc 64, 1361 (PASA), Newton et al 1989 Nucl Acids Res 17, 2503 (ARMS), Wu et al 1989 PNAS 86, 2757 (ASPCR)) both make use of 3'-end primer specificity. The theory here is that in two complementary reactions- one contains a primer specific for the normal allele and the other contains one for the mutant allele (both have a common 2nd primer) so that one PCR primer perfectly matches one allelic variant of the target but is mismatched to the other. The prior art with respect to the invention discloses methods wherein a given primer will bind e.g. in the presence of an MRSA sample because of its location within the SCCmec region but not in the mere presence of an MSSA sample (which lacks the SCC*mec* region.

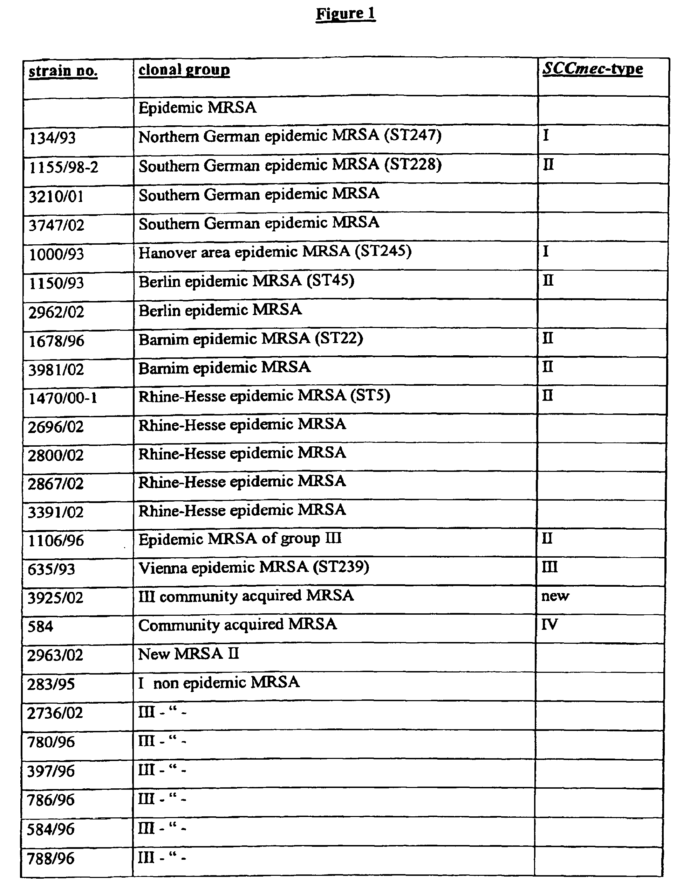

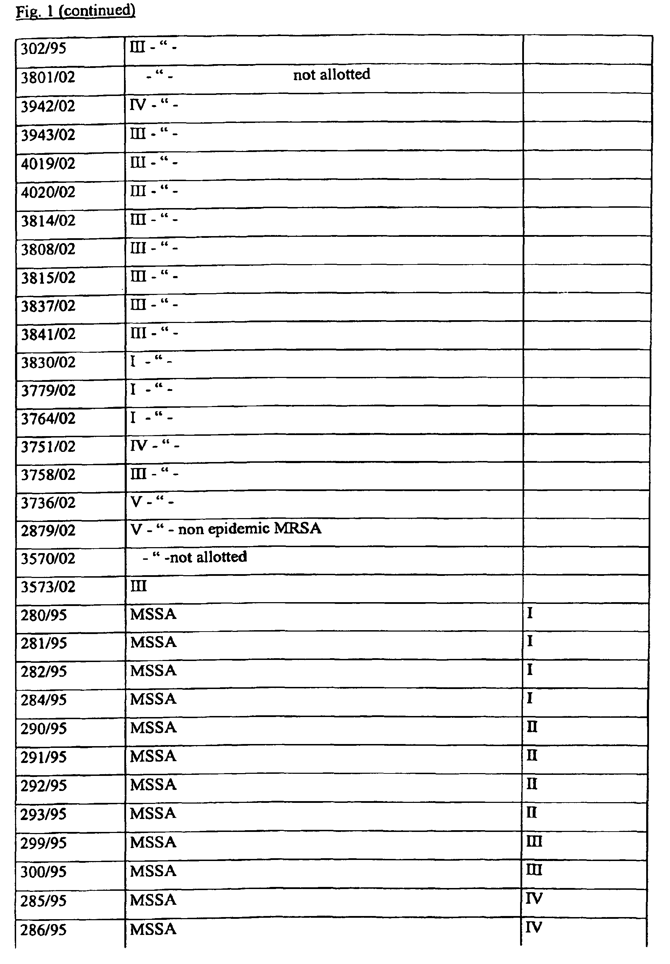

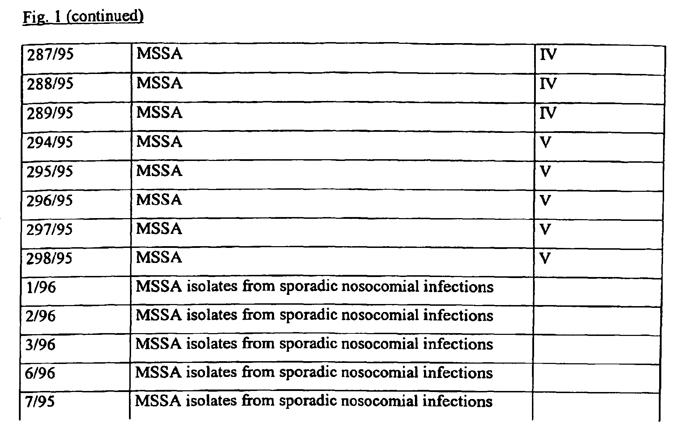

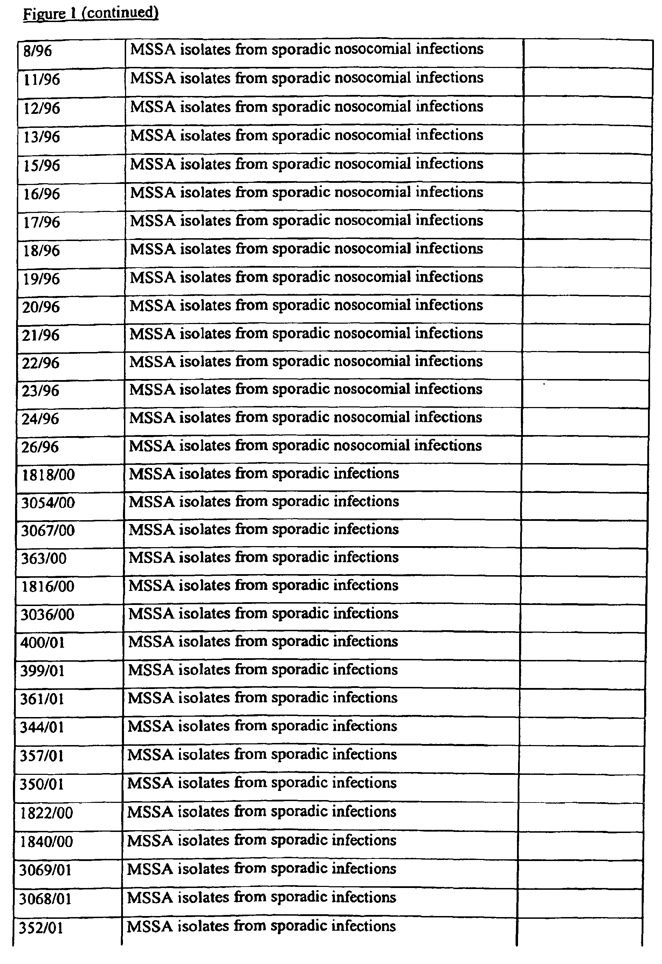

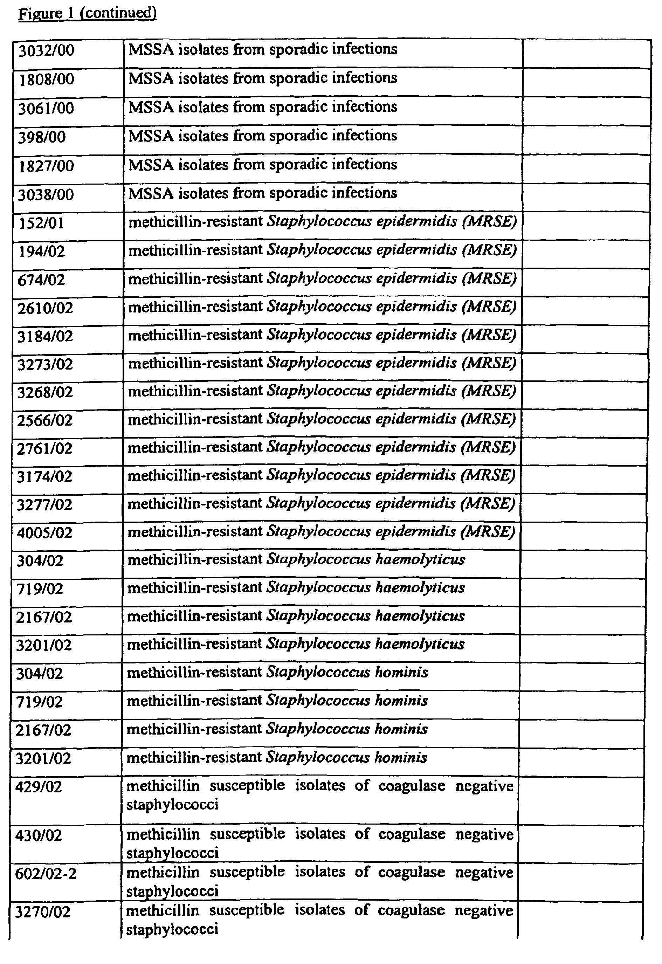

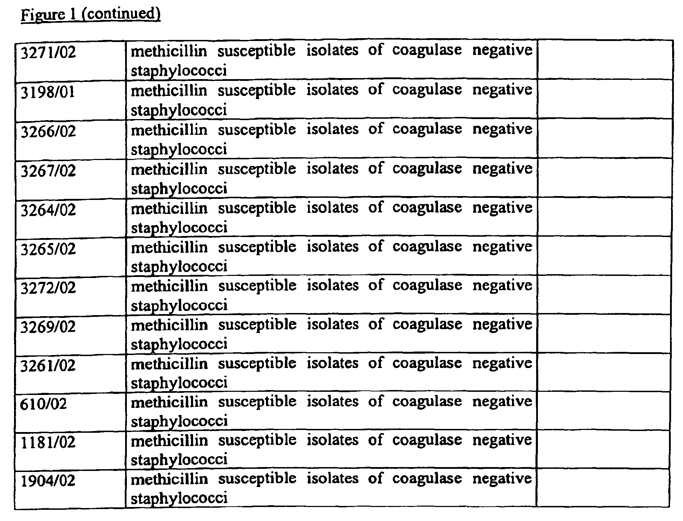

In this case the inventors have astonishingly found out that under certain PCR conditions one can place one or more primers in such way that the 3' end lies with the CSDNA (present for example in MSSA and MRSA) and it is still possible to obtain a reliable experimental result with respect to the question of whether or not MRSA strains are contained within a sample that may comprise both MRSA as well as CNS strains. In fact, to date the inventors have investigated a total of 167 (one hundred sixty seven) strains. In all case detection was correct. The inventors have investigated a total of 45 MRSA strains, 60 methicillin sensitive *Staphylococcus aureus* strains (MSSA) and 62 coagulase negative strains (CNS). Figures 1 to 9 give a detailed representation of all strains analyzed. Surprisingly, in all cases the method according to the invention performed well.

Herein, the sequence specific amplification may accomplished by means of the ligase chain reaction method, the rolling circle amplification method, the PCR amplification method or other amplification methods known to those skilled in the art. In a preferred embodiment of the invention it is done by means of the PCR amplification method.

In such a PCR amplification reaction the following steps are performed a) combining the sample containing the target region, here the nucleic acid molecule according to the invention, with at least on nucleotide triphosphate, a thermally stable polymerase, a buffer and two sequence specific primers, b) exposing the reaction mixture to at least one temperature cycle including at least a high temperature denaturation phase and a lower temperature extension phase, and thereby producing at least a partially amplified product. Ideally the cycles are repeated and thus a larger quantity of the product of desire is created.

In this method according to the invention it is preferred that the primers according to the invention comprises preferably between 14 and 28 nucleotides more preferably between 15 and 25 nucleotide most preferably between 16 and 22 nucleotides for each nucleic acid moiety. It is essential in this context that the primers are of sufficient length to bind the target nucleic acid molecule with sufficient stringency. The method according to the invention is special with respect to the primers as said first primer relies on a well tuned ratio between binding capabilities and annealing temperature. Said first nucleic acid primer has a sequence according to SEQ ID NO. 1 or SEQ ID NO. 5, the 5' end of which lies within the SCCmec region of the bacterial chromosome and 3' end of which lies in the chromosomal surrounding DNA (CSDNA). Within a particular temperature range the said first primers according to the invention will not lead to false products.

PNA-DNA hybrid oligonucleotides (see Finn, P. J. et al., N.A.R. 24, 3357-3363 (1996), Koch, T. et al., Tetrahedron Letters 36, 6933-6936 (1995), Stetsenko, D. A. et al., Tetrahedron letters 37, 3571-3574 (1996), Bergmann, F et al., Tetrahedron Letters 36 6823-6826 (1995) and Will, D. W. et al., Tetrahedron 51, 12069-12082 (1995)) are also considered as primers for the process of the present invention.

As a thermally stable polymerase, a DNA polymerase may be selected from the group comprising *Taq* DNA polymerase, *Tth* DNA polymerase or Klentaq (Taq DNA polymerase (-exo5'-3'), Korolev et al., (1995) Proc. Natl. Acad. Sci. USA 92, 9246-9268. The use of Taq DNA polymerase in the method of the present invention is especially preferred. One skilled in the art will recognize that numerous different thermally stable polymerases may be used.

Alternatively as a thermally stable polymerase, a DNA polymerase which has a decreased discrimination against labelled nucleotides may be used. This makes it possible to easily incorporate nucleotides that are labelled.

The number of thermal cycles may range from about 10 to about 45 depending on the amount of template DNA and its purity. As the availability of the nucleic acid according to the invention is high in the method according to the invention the cycle period may be short.

Routinely, cycling consists of (i) a denaturing cycle, (ii) an annealing cycle and (iii) an extension cycle. Alternatively, only two cycles may be applied, (i) a denaturing cycle and (ii) an annealing and extension cycle.

Preferably the denaturing cycle is performed at between 100°C and 85°C, more preferably at between 98°C and 90°C, most preferably at between 96°C and 92°C.

Preferably the annealing cycle is performed at between 72°C and 45°C, more preferably at between 60°C and 50°C, most preferably at between 60°C and 55°C.

Preferably the extension cycle is performed at between 80°C and 50°C, more preferably at between 75°C and 60°C, most preferably at between 73°C and 68°C.

Preferably the denaturing cycle is performed for 3 minutes, more preferably for 30 seconds.

Preferably the annealing cycle is performed for 4 minutes, more preferably for 30 seconds.

Preferably the extension cycle is performed for 3 minutes, more preferably for 30 seconds, however the extension time vary depending on the length of the template, in particular the extension time may be raised if the template length increases.

Buffers components which can be used can include, but are not limited to, Tris-HCl at a pH of about 7.0 to 10 and concentration of about 2 to 60 mM, ammonium sulfate at a concentration of about 10-20 mM, preferably 15 mM, MgCl₂ at a concentration of about 1 to 10 mM, and optionally, about 0.05 mM mercaptoethanol, about 0.28% Tween® 20 and/or about 0.02% Nonidet® 40.

Nucleotide triphosphates are preferably deoxynucleotides and can be selected from, but are not limited to, dGTP, dATP, dTTP and dCTP. In addition, derivatives of deoxynucleotides, which are defined as those deoxynucleotides which are capable of being incorperated by a thermally stable DNA polymerase into nascent DNA molecules synthesized in the thermal cycling reaction, can also be used according to the invention. Such derivatives include, but are not limited to thionucleotides, 7-deaza-2'-dGTP, 7-deaza-2'-dATP as well as deoxyinosine triphosphate which may also be used as a replacement deoxynucleotide for dATP, dGTP, dTTP or dCTP. The above mentioned deoxynucleotides and derivatives thereof are preferably used at a concentration of about 50 µM to about 4 mM.

In a preferred embodiment one or more of the nucleotides incorporated are labeled. For example, single and differential labels may consist of the group comprising enzymes such as β-galactosidase, alkaline phosphatase and peroxidase, enzyme substrates, coenzymes, dyes, chromophores, fluorescent, chemiluminescent and bioluminescent labels such as FITC, Cy5, Cy5.5, Cy7, Texas-Red and IRD40(Chen et al. (1993), J. Chromatog. A 652: 355-360 and Kambara et al. (1992), Electrophoresis 13: 542-546), ligands or haptens such as biotin, and radioactive isotopes such as ³H, ³⁵S, ³²P ¹²⁵I and ¹⁴C.

In one embodiment of the method of the invention, the nucleic acid molecule to be amplified can be present in the form of total genomic DNA. Preferably, the genomic DNA has a length greater than or equal to 2 kb. Generally all forms of template may be used, *e.g.* purified nucleic acids, *i.e.* nucleic acids where one fraction may be enriched or not, one example being plasmid DNA the other purified genomic DNA. The method may also be performed and this preferred by simply adding *Staphylococcus aureus* cells or rather the sample to be analyzed to the PCR reaction. The sample may stem from overnight cultures or directly from the patient.

In a particularly preferred embodiment the PCR reactions are performed with Ready-to-go PCR beads (Amersham Pharmacia Biotech, Freiburg, Germany) in a 25 µl reaction containing approximately 10 ng of template DNA and 2.5 pmol of each primer. Initial denaturation at 94°C for 3 min is followed by 30 cycles of amplification with 94°C for 30 sec, annealing at 55°C for 30 sec and extension at 72°C for 30 sec (except for the final cycle with an extension step of 4 min).

PCR products may be labelled by either using digoxigenin-labelling for hybridization to nitrocellulose bound capture probes or by random primed labelling with fluorescein and use of the Flourescein High Prime* kit (Roche, Mannheim, Germany) in case of microarrays. Other labelling and detection procedures are known to those skilled in the art.

The second nucleic acid primer present in the reaction lies in the downstream CSDNA and serves as a downstream opposing primer to said first primer. Numerous primers are possible it is essential that the one or more primers chosen fulfils the following prerequisites: (i) it has an annealing temperature that is similar to that of said first primer and it has a sequence that is sufficiently conserved in all strains to ensure that all strains are amplified which need to be detected. Depending on the geographical region in the world and the diagnostic question that must be answered various different second downstream primers may be chosen.

The inventors have been able to identify downstream primers which make it possible to adopt the method according to the invention to various different detection methods. In cases where the detection is done using an array or a filter it may be advantageous to have small amplification products whereas in cases where detection is to be done by means of an agarose gel which is not as suited for a hospital environment as outlined above it is better to have amplification products which are between about 100 bp and 800 bp in size. In a preferred embodiment the downstream primer and the upstream primer create a product of about 600 bp in size, in a very preferred embodiment the size of the product is under 200 bp, in a particularly preferred embodiment the size of the product is under 100 bp. In a particularly preferred embodiment the downstream primer may be chosen from the group comprising, (SEQ ID NO. 2) 5'-AAC GTT TAG GCC CAT ACA CCA-3' corresponding to pos. 39256-39236 in AB033763, (SEQ ID NO. 3) 5'-ATG ACA TTC CCA CAT CAA ATG-3' corresponding to pos. 38942-38922 in AB033767 and (SEQ ID NO. 4) 5'-ACA TCA AAT GAT GCG GGT-3' corresponding to pos. 38931-38914 in AB033767.

In a very preferred embodiment of the invention said second nucleic acid primer is able to bind a sequence according to SEQ ID No. 8 in an amplification reaction comprising between 1 mMol and 3 mMol MgCl₂, at an annealing temperature of between 45°C and 55°C.

In a particularly preferred embodiment of the invention said second nucleic acid primer is chosen from the group comprising SEQ ID NO.2, 3 and 4.

In a particularly preferred embodiment of the invention said first nucleic acid primer consist of a mixture of primers with the following sequences SEQ ID NO. 1 and/or 5.

The detection of the product may done using an agarose gel or various other methods known to those skilled in the art. In a preferred embodiment of the invention the detection in step (ii) of the method according to the invention is performed with the aid of one or more sequence specific hybridisation probes in hybridization reaction wherein, the probes are able to bind the amplification product produced in step (i) under stringent conditions. This has numerous advantageous. Agarose gels and other methods are not suited for a hospital environment. Whereas array test need less manual care and can those be performed in a hospital. Additionally fewer machines are required.

Hybridization probes act by forming selectively duplex molecules with complementary stretches of a sequence of a gene or a sample or a cDNA in our case an amplification product. The selectivity of the process can be controlled by varying the conditions of hybridization.

Stringent conditions for the hybridization will be used, *e.g.* low salt in the range of 0.02 M to 0.15 M salt and/or high temperatures in the range from 50°C degrees centigrade to 70°C degrees centigrade. Stringency can be further improved by the addition of formamide to the hybridization solution. The use of stringent conditions which means that only little mismatch or a complete match will lead to a hybridization product is important for the invention. Thus, as used herein stringent hybridization conditions are those where between 0.02 M to 0.15 M salt and/or high temperatures in the range from 50°C degrees centigrade to 70°C degrees centigrade are applied and where long probes are used, *i.e.* probes that are longer than 30 nucleotides.

The use of highly stringent conditions or conditions of "high stringency" means that only very little mismatch or a complete match will lead to a hybridization product. Thus, as used herein highly stringent hybridization conditions are those where between 0.02 - 0.3 M salt and 65°C degrees centigrade are applied for about 5 to 18 hours of hybridization time and additionally, the sample filters are washed twice for about 15 minutes each at between 60°C - 65°C degrees centigrade, wherein the first washing fluid contains about 0.1 M salt (NaCl and/or Sodium Citrate) and the second contains only about 0.02 M salt (NaCl and/or Sodium Citrate).

In a preferred embodiment the following conditions are considered to be highly stringent:

Hybridisation in a buffer containing 2 x SSC (0.03 M Sodium Citrate, 0.3 M NaCl) at 65°C - 68°C degrees centigrade for 12 hours, followed by a washing step for 15 minutes in 0.5 x SSC, 0.1% SDS, and a washing step for 15 minutes at 65°C degrees centigrade in 0.1 x SSC, 0.1% SDS.

In a very preferred embodiment hybridization is performed in an H1-buffer (5x puffer H1: 0.55 g Na₂HPO₄, 0.1 g NaHₐPO₄, 0.625 ml 20 x SSC, 0.045 g EDTA, 0.8 g SPS, 0.25 ml Aq. bidest), at a temperature between 35°C and 45°C most preferentially at 42°C for 1 to 8 hours most preferentially for 4 hours. Washing is performed in 1 X SSC to 3 X SSC, most preferentially in 2 X SSC with 0.1% SDS to about 3% SDS, most preferentially 0.5% SDS at about room temperature.

Less stringent hybridization conditions, e.g. 0.15 M salt - 1 M salt and/or temperatures from 22°C degrees centigrade to 56°C degrees centigrade lead to unspecific results.

Unspecific hybridization products are removed by washing the reaction products repeatedly in 2 x SSC solution and increasing the temperature.

In one embodiment the invention the detection step is performed on a dry rapid test. Here the method is performed using a chromatographic material that comprises a sample reception zone, a separation zone including a binding area, in which one or more sequence-specific nucleic acids are immobilized, and a zone which absorbs a liquid downstream of the separation zone including a binding area. In this embodiment, the sequence-specific nucleic acid probes are immobilized via a polymer linker. The method comprises the following steps i) denaturing, in the case of double-stranded nucleic acids, the nucleic acid to be detected and then neutralizing it, ii) applying the nucleic acid to be detected to the sample reception zone in a run buffer which contains mildly denaturing agents, iii) the nucleic acid moving from the sample reception zone in direction of the liquid absorbing zone, (iv) contacting the nucleic acid to be detected in the binding area of the separation path with the sequence-specific nucleic acid probe and hybridizing it with the sequence-specific nucleic acid probe, v) detecting the nucleic acid or the hybridization of the nucleic acid with the sequence specific nucleic acid probe via a label that is attached to the nucleic acid to be detected or via the detection of a label of the nucleic acid double strand (WO 03/039703).

The invention also encompasses a kit for performing the method according to the invention comprising a least a first nucleic acid primer according to the invention. Such a kit may additionally comprise one or more enzymes, nucleotides, buffers and other reagents which are necessary or desired for performing the method according to the invention. Said kit may also comprise reagents and materials for performing the detection according to the invention. In the accompanying figures and tables,
**Figure 1:** shows a list of isolates investigated using the method according to the invention.

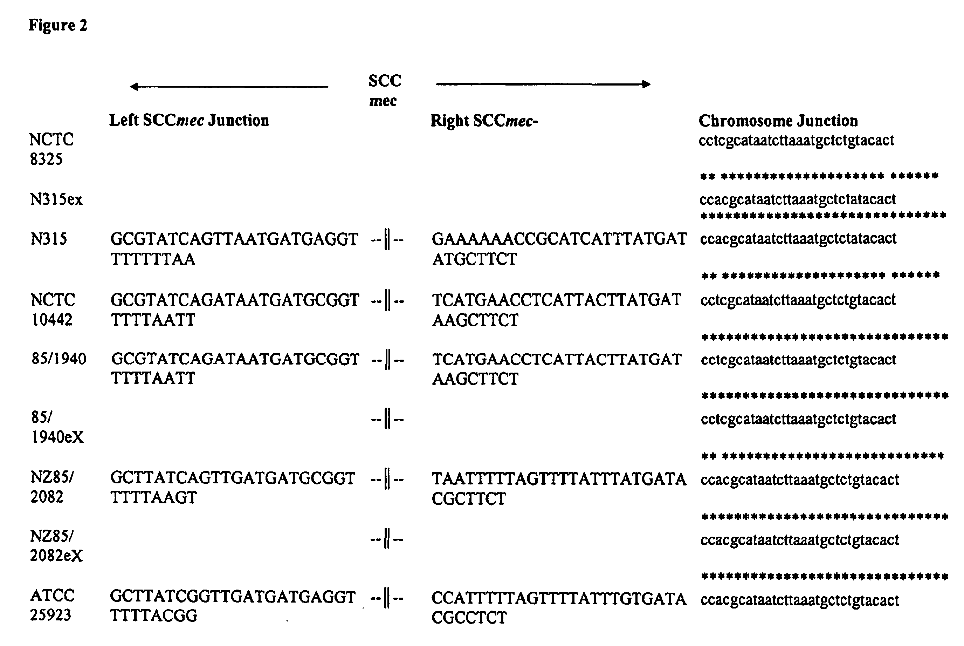

**Figure 2:** shows published DNA-sequences at the right junction of until now described SCCmec cassettes in methicillin resistant *Staphylococcus aureus.*

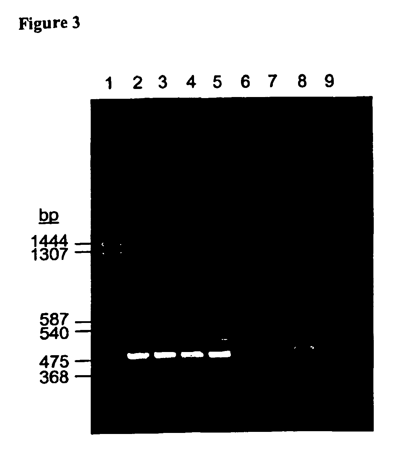

**Figure 3:** shows an agarose gel with amplimers of PCR for SEQ ID NO. 1 and SEQ ID NO. 2. Fig. 3 depicts in lane 1: 10mol. mass standards, in lane 2: strain 134/93; annealing temperature 50°C, in lane 3: strain 131/98; annealing temperature 50°C, in lane 4: strain 1678/98; annealing temperature 50°C, in lane 5: strain 1150/93; annealing temperature 50°C, in lane 6: strain 134/93; annealing temperature 55°C, in lane 7: strain 131/98; annealing temperature 55°C, in lane 8: strain 1678/98; annealing temperature 55°C and in lane 9: strain 1150/93; annealing temperature 55°C. When PCR is performed by use of primers corresponding to SEQ ID No. 1 and SEQ ID No. 2 the "unspecific" PCR-products of 480 bp is avoided.

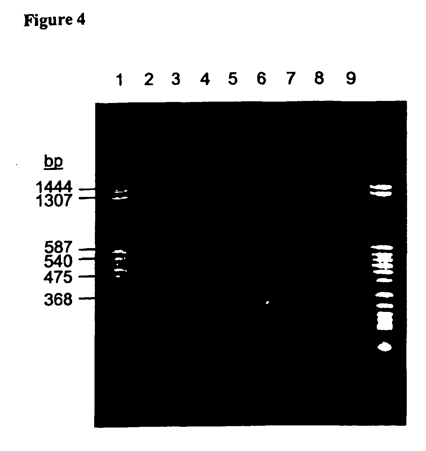

**Figure 4:** shows an agarose gel with amplimers of PCR for SEQ ID NO. **1 and** SEQ ID NO. 4: Fig. 4 depicts the method according to the invention lane 1, 10: mol. mass standards, lane 2: Strain 134/93; lane 3: 131/98 (SCCmecI), lane 4: Strain 1678/98; 5: 3391/02 (SCCmecII), lane 6: Strain 635/93 (SCCmecIII), lane 7: Strain 1150/93 (SCCmecII), lane 8: Strain 3925/03 (new SCCmec), lane 9: Strain 584/02 (SCCmecIV). It can be seen that the method gives positive results for all SCCmec types.
**Figure 5:** shows primers for specific detection of MRSA according to patent PCT WO 02/099034 A2 as shown in table 1: As can bee seen specific primers have to be introduced newly according to evolution of MRSA. That means this system has to be continuously adapted to the actual epidemiological situation.

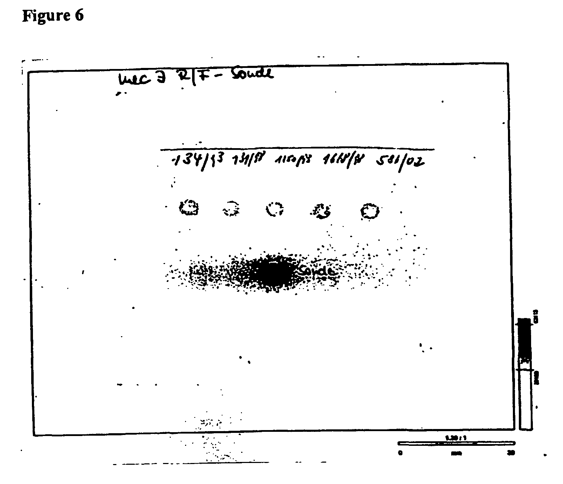

**Figure 6:** shows the results obtained by applying the method according to the invention. Herein, an example for detection of the overlapping nucleotide sequence at the right junction of *SCCmec* gene cassettes and the chromosome ORFX sequence by use of a macroarray on nitrocellulose. PCR was performed with primers according to SEQ ID NO 1 and SEQ ID NO 2 with digoxigenin labelling; Hybridization was done with an ORFX specific capture probe 5'-GAT GCG GGT TGT GTT AAT TGA, immobilized with a 5'-linked polymer. Detection of the hybridized probe was performed with alkaline phosphatase.

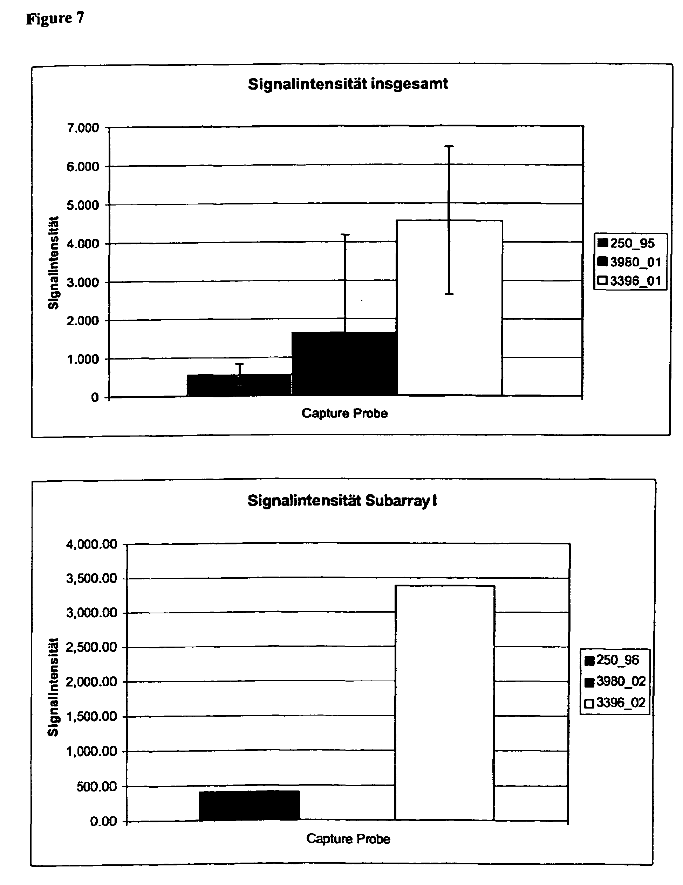

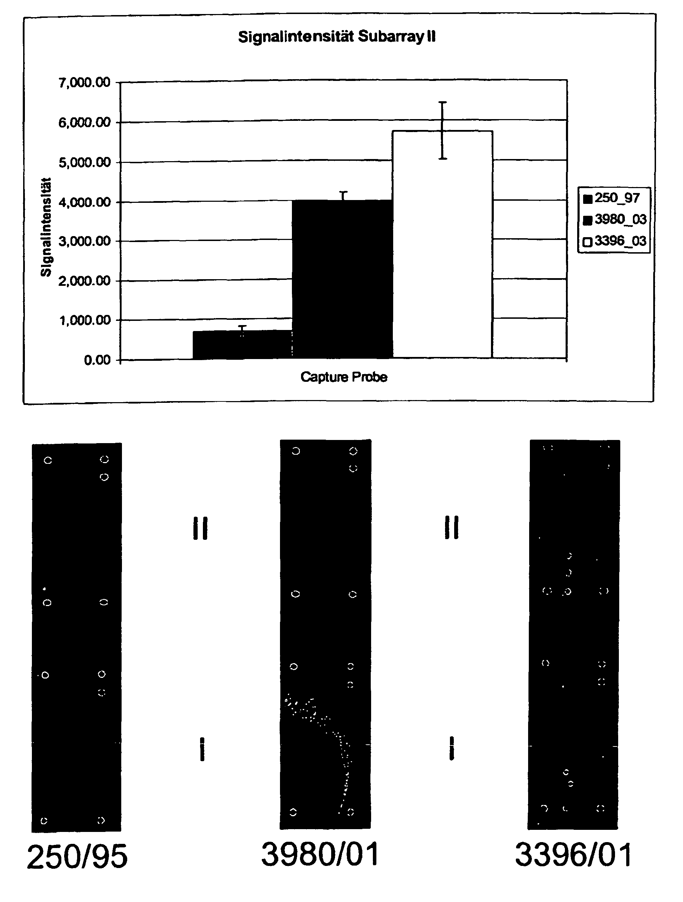

**Figure 7:** shows the application of the method according to the invention using array technology. It demonstrates the specific detection of the overlapping nucleotide sequence at the right junction of *SCCmec* gene cassettes and the chromosomal ORFX sequence. Amplification was performed using SEQ ID NO: 1 and SEQ ID NO 4 an amplification product of 78 bp was generated. Subsequent labelling was performed with random primer PCR and fluorescein. Hybridization was done with an ORFX specific probe 5'-GAT GCG GGT TGT GTT AAT TGA specific for the strains 250/95 MSSA; 3980/01, 3396/01 MRSA.

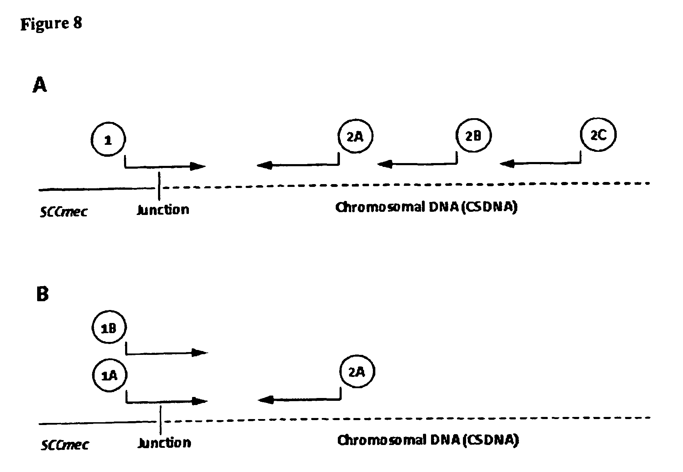

**Figure 8:** shows the method according to the invention. Here, as shown in (A) the invention provides for a method of detecting a methicillin resistant *Staphylococcus aureus* strain in a sample, said strain comprising a chromosomal SCC*mec* insert containing a *mec*A gene, wherein the method comprises the steps of: (i) performing a sequence specific amplification reaction wherein, the amplification reaction comprises one or more first nucleic acid primers, the 5' end of which lies within the SCC*mec* region of the bacterial chromosome and 3' end of which lies in the chromosomal surrounding DNA (CSDNA), here shown as at "1", least a second nucleic acid primer is present in the reaction which lies in the downstream CSDNA and which serves as a downstream opposing primer to said first primer, here shown as "2", and (ii) detecting the presence or absence of an amplification product from step (i). As can be seen from the figure the 3'end of the first nucleic acid primer lies in the CSDNA. One skilled in the art would expect that also those samples not comprising a MRSA strain (only MSSA for example) would give a positive result when analyzed. However, as outlined above this is not the case. As outlined for example in "MRCPath preparation course 1999-00 Mutation Detection Systems (http://www.ich.ucl.ac.uk/cmgs/arms99.htm)" an ARMS primer is designed so that the nucleotide distinguishing between the different alleles is at the 3' terminal base of the primer (when it is specific to the template it will amplify and when it is differs it will not). Thus, one skilled in the art will always chose the position of the 3'end of the primer to lie within the allele one wishes to differentiate. Figure 8 shows in section (B) that in one embodiment of the invention said first nucleic acid primer according to the invention may also consist of a mixture of primers of equal or varying length with varying sequences. All of said first nucleic acid primers however have in common that the 3'-end lies with the CSDNA region and the 5'-end lies in the *SCCmec* region.

**Table 2: Listing of MSSA, reference strains used as clonal groups for testing the method according to the invention.**

| Strain no. | clonal group |
|---|---|
| 280/95, 281/95, 282/95, 284/95 | I |
| 290/95, 291/95, 292/96, 293/95 | II |
| 299/95,300/95 | III |
| 285/95, 286/95, 287/95, 288/95, 289/95 | IV |
| 294/95, 295/95, 296/95, 297/95, 298/95 | V |

**Table 3:** Listing of MSSA isolates from sporadic nosocomial infections tested using the method according to the invention.
1/96, 2/96, 3/96, 6/96, 7/96, 8/96, 11/96, 12/96, 13/96, 15/96, 16/96, 17/96, 18/96, 19/96, 20/96, 21/96, 22/96, 23/96, 24/96, 26/96

**Table 4:** Listing of MSSA isolates from sporadic infections in the community tested using the method according to the invention.
1818/00, 3054/00, 3067/00, 363/01, 1816/00, 3036/00, 400/01, 399/01, 361/01, 344/01, 357/01, 350/01, 1822/00, 1840/00, 3069/00, 3068/00, 352/01, 3032/00, 1808/00, 3061/00, 398/00, 1827/00, 3038/00

**Table 5:** Listing of methicillin-resistant *Staphylococcus epidermidis* (MRSE) tested using the method according to the invention.
152/01, 194/02, 674/02, 2610/01, , 3184/02, 3273/02, 3268/02, 194/02, 2566/02, 2761/02, 3174/02,3277/02,4005/02

**Table 6:** Listing of methicillin-resistant *Staphylococcus haemolyticus* tested using the method according to the invention.
304/02, 719/02, 2167/02, 3201/02

**Table 7:** Listing of methicillin-resistant *Staphylococcus hominis* tested using the method according to the invention.
304/02, 719/02, 2167/02, 3201/02

**Table 8:** Listing of methicillin susceptible isolates of coagulase negative staphylococci tested using the method according to the invention.
S. epidermidis, 429/02, 430/02, 602/02-2, 3270/02, 3271/02, 3198/01, 3266/02, 3267/02, 3264/02, 3265/02, 3272/02, S. capitis 3269/02, S. lugdunensis 3261/02, S. warneri 610/02, 584/02-1, 1181/02, S. sciurii 1904/02

### Example 1

Method according to the invention. The *mec*A gene of *S. aureus* is incorporated in chromosomal cassettes with a more or less homologous sequence downstream *mecA* (Figure 2). For covering this part the following primer sequences were chosen which allow detection of the different cassette types known:

Primer for the right junction of SCCmec's and first two base pairs of ORFX (CSDNA) primer rjmecf (SEQ ID NO.1): 5'-TTA TGA TAA GCT TCT CC-3'. The ORF-X sequence adjacent to SCCmec's is rather homologous among different clonal lineages of *S. aureus.* As outlined above the unexpected results are obtained primarily due to the location and positioning of this nucleic acid wherein the 5' end lies within the SCC*mec* region of the bacterial chromosome and 3' end of which lies in the chromosomal surrounding DNA (CSDNA).

Numerous downstream primers are possible. Three primer sequences have been chosen as reverse primers. They make various applications of the method according to the invention possible. In one embodiment detection of the product is simply done by means of an agarose gel. The inventors have identified a primer position which gives a size fragment of 403 bp. In this embodiment of the invention the downstream nucleic acid according to the invention may be (SEQ ID NO. 2) 5'-AAC GTT TAG GCC CAT ACA CCA-3' corresponding to pos. 39256-39236 in AB033763.

In one embodiment detection of the product is done by means of array technology. The inventors have identified a primer position which gives a size fragment of 89 bp. In this embodiment of the invention the downstream nucleic acid according to the invention may be (SEQ ID NO. 3) 5'-ATG ACA TTC CCA CAT CAA ATG-3' relating to pos. 38942-38922 in AB033767. Alternatively it may be (SEQ ID NO. 4) 5'-ACA TCA AAT GAT GCG GGT-3' corresponding to pos. 38931-38914 in AB033767. The latter results in a product of 78 bp.

### Amplification

Whole cellular DNA is prepared from cells derived from an overnight culture on blood agar plates from about 10 single colonies with the DNeasy Tissue kit from Qiagen, Hilden, Germany). Single PCR reactions were performed with Ready-to-go-PCR beads (Amersham Pharmacia Biotech, Freiburg, Germany) in a 25 µl reaction containing approximately 10 ng of template DNA and 2.5 pmol of each primer.

For primer pairs SEQ ID NO. 1 and SEQ ID NO. 2 the initial denaturation at 94°C for 3 min was followed by 30 cycles of amplification with 94°C for 30 sec, annealing at 55°C for 30 sec and extension at 72°C for 30 sec (final cycle with an extension step of 4 min). Products were analysed on a 1.5% agarose gel and further controlled by sequencing. A higher amplimer quantity could be obtained by lowering annealing temperature to 50°C.

For primer pairs SEQ ID NO. 1 and SEQ ID NO. 3 or SEQ ID NO. 1 and SEQ ID NO. 4 the initial denaturation at 95°C for I min, was followed by 5 cycles at 95°C for 30 sec, 45°C for 60 sec, 72°C for 20 sec, 25 cycles at 95°C for 30 sec, 45°C for 30 sec, 72°C for 15 sec. A final cycle followed at 95°C for 30 sec, 45°C for 30 sec, 72°C for 50 sec.

Examples are shown in Figure 3 using primer pair SEQ ID NO. 1 and SEQ ID NO. 2 both at an annealing temperature of 55°C [lanes 2-5] and 50°C [lanes 6-9].

Examples are shown in Figure 4 using primer pair SEQ ID NO. 1 and SEQ IDNO. 4 at an annealing temperature of 45°C. Sequencing of the obtained amplimers gave results corresponding to sequences for this region of SCCmec's I and II from data banks (AB033763, D86934).

## Claims

1. Method of detecting a methicillin resistant *Staphylococcus aureus* strain in a sample, said strain comprising a chromosomal staphylococcal cassette chromosome *mec* (SCC*mec*) insert containing a *mec*A gene, wherein the *Staphylococcus aureus* strain may be chosen from the group comprising *Staphylococcus aureus* type I, type II, type III, type IV and wherein the method comprises the steps of:
(i) performing a sequence specific amplification reaction wherein, the amplification reaction comprises one or more first nucleic acid primers, wherein said first nucleic acid primer has a sequence according to SEQ ID NO. 1 or SEQ ID NO. 5, the 5' end of which lies within the SCC*mec* region of the bacterial chromosome and 3' end of which lies in the chromosomal surrounding DNA (CSDNA), at least a second nucleic acid primer is present in the reaction which lies in the downstream CSDNA and which serves as a downstream opposing primer to said first primer, and
(ii) detecting the presence or absence of an amplification product from step (i).

2. Method according to claim 1 wherein, said second nucleic acid primer is able to bind a sequence according to SEQ ID No. 8 in an amplification reaction comprising between 1 mMol and 3 mMol MgCl₂, at an annealing temperature of between 45°C and 55°C.

3. Method according to claim 2 wherein, said second nucleic acid primer is chosen from the group of SEQ ID NO. 2, 3 and 4.

4. Method according to any of the preceding claims wherein, said first nucleic acid primer consist of a mixture of primers with the following sequences SEQ ID NO. 1 and SEQ ID NO. 5.

5. Method according to any of the preceding claims wherein, the detection in step (ii) is performed with the aid of one or more sequence specific hybridisation probes in a hybridization reaction wherein, the probes are able to bind the amplification product produced in step (i) under stringent conditions.

6. Method according to claim 5 wherein, the hybridization is performed with a chromatographic material that comprises (i) a sample reception zone, (ii) a separation zone including a binding area, in which one or more sequence-specific nucleic acids are immobilized, (ii) and a zone which absorbs a liquid downstream of the separation zone including a binding area, (iii) and the sequence-specific nucleic acid probes are immobilized via a polymer linker and wherein the method comprises the following steps:
a) denaturing, in the case of double-stranded nucleic acids, the nucleic acid to be detected and then neutralizing it,
b) applying the nucleic acid to be detected to the sample reception zone in a run buffer which contains mildly denaturing agents,
c) the nucleic acid moving from the sample reception zone in direction of the liquid absorbing zone,
d) contacting the nucleic acid to be detected in the binding area of the separation path with the sequence-specific nucleic acid probe and hybridizing it with the sequence-specific nucleic acid probe,
e) detecting the nucleic acid or the hybridization of the nucleic acid with the sequence specific nucleic acid probe via a label that is attached to the nucleic acid to be detected or via the detection of a label of the nucleic acid double strand.

7. Kit for performing a reaction according to one of the preceding claims, comprising a nucleic acid primer according to SEQ ID NO.1 or according to SEQ ID NO. 5 or comprising both.

## Patentansprüche

1. Verfahren zum Nachweis eines Methicillinilin-resistenten *Staphylococcus aureaus* Stammes in einer Probe, wobei der Stamm ein chromosomales Staphylokokken *mec* (SCC*mec*) Insert, welches wiederum ein *mec*A-Gen trägt, umfaßt, wobei der *Staphylococcus aureaus-*Stamm ausgewählt sein kann, aus der Gruppe umfassend *Staphylococcus aureaus* Typ I, Typ II, Typ III, Typ IV und wobei das Verfahren die Schritte:
(i) Durchführung einer sequenzspezifischen Amplifikationsreaktion, wobei die Amplifikationsreaktion einen oder mehrere erste Nukleinsäureprimer umfaßt, wobei jener erster Nukleinsäureprimer eine Sequenz gemäß SEQ ID Nr. 1 oder SEQ ID Nr. 5 hat, dessen 5'-Ende innerhalb der SCC*mec*-Region des bakteriellen Chromosoms liegt und dessen 3'-Ende in der umliegenden chromosomalen DNA (CSDNA) liegt, mindestens ein zweiter Nukleinsäureprimer in der Reaktion vorhanden ist, der downstream in der CSDNA liegt und als Gegenprimer zu jenem ersten Primer fungiert; und
(ii) Nachweis der Anwesenheit oder Abwesenheit eines Amplifikationsproduktes aus Schritt (i), umfaßt.

2. Verfahren nach Anspruch 1, wobei jener zweite Nukleinsäureprimer in der Lage ist, eine Sequenz gemäß SEQ ID Nr. 8 in einer Amplifikationsreaktion umfassend zwischen 1 mMol und 3 mMol MgCl₂ bei einer Annealing-temperatur von zwischen 45°C und 55°C zu binden.

3. Verfahren nach Anspruch 2, wobei jener zweite Nukleinsäureprimer aus der Gruppe von SEQ ID Nr. 2, 3 und 4 ausgewählt ist.

4. Verfahren nach einem der vorangehenden Ansprüche, wobei jener erste Nukleinsäureprimer aus einem Gemisch von Primem mit den folgenden Sequenzen, SEQ ID Nr. 1 und SEQ ID Nr. 5, besteht.

5. Verfahren nach einem der vorangehenden Ansprüche, wobei der Nachweisschritt in Schritt 2 mit Hilfe einer oder mehrerer Sequenzhybridisierungsproben in einer Hybridisierungsreaktion stattfindet und wobei die Proben in der Lage sind, das Amplifikationsprodukt, welches in Schritt (i) gebildet wurde, unter stringenten Bedingungen zu binden.

6. Verfahren nach Anspruch 5, wobei die Hybridisierung mit einem chromatographischen Material durchgeführt wird, welches (i) eine Probenaufnahmezone, (ii) eine Trennzone mit Bindungsbereich, in welchem eine oder mehrere sequenzspezifische Nukleinsäuresonden immobilisiert sind, und (iii) die sequenzspezifischen Nukleinsäuresonden über einen Polymerlinker immobilisiert sind und wobei das Verfahren die folgenden Schritte umfaßt:
a) die nachzuweisende Nukleinsäure wird im Falle doppelsträngiger Nukleinsäuren denaturiert und anschließend neutralisiert,
b) die nachzuweisende Nukleinsäure wird auf die Probenaufnahmezone in einem Laufpuffer, welcher mild denaturierende Agenzien enthält, aufgetragen,
c) die nachzuweisende Nukleinsäure bewegt sich von der Probenaufnahmezone in Richtung flüssigkeitabsorbierende Zone,
d) die nachzuweisende Nukleinsäure wird im Bindungsbereich der Trennstrecke mit der sequenzspezifischen Nukleinsäuresonde in Kontakt gebracht und hybridisiert die sequenzspezifische Nukleinsäuresonde,
e) die nachzuweisende Nukleinsäure bzw. die Hybridisierung der nachzuweisenden Nukleinsäure an die sequenzspezifische Nukleinsäuresonde wird detektiert über eine Markierung, die an der nachzuweisenden Nukleinsäure angebracht ist oder über die Detektion einer Markierung des Nukleinsäure-Doppelstranges.

7. Kit zur Durchführung einer Reaktion nach einem der vorangehenden Ansprüche umfassend eine Nukleinsäure nach SEQ ID Nr. 1, nach SEQ ID Nr. 5 oder umfassend beide.

## Revendications

1. Procédé de détection d'une souche *Staphylococcus aureus* résistante à la méthicilline dans un échantillon, ladite souche comprenant une insertion de la cassette chromosomique staphylococcique mec(SCCmec) contenant un gène *mec*A, dans lequel la souche *Staphylococcus aureus* peut être choisie parmi les *Staphylococcus aureus* de type I, type II, type III, type IV et dans lequel le procédé comprend les étapes consistant à :
(i) réaliser une réaction d'amplification spécifique d'une séquence dans laquelle la réaction d'amplification comprend une ou plusieurs premières amorces d'acides nucléiques, dans laquelle ladite première amorce d'acides nucléiques présente une séquence selon SEQ ID NO.1 ou SEQ ID NO.5, dont l'extrémité 5' se situe dans la région SCCmec du chromosome bactérien et dont l'extrémité 3' se situe dans l'ADN environnant chromosomique (CSDNA), au moins une seconde amorce d'acides nucléiques est présente dans la réaction qui se situe en aval de CSDNA et qui sert d'amorce opposée en aval par rapport à ladite première amorce, et
(ii) détecter la présence ou l'absence d'un produit d'amplification de l'étape (i).

2. Procédé selon la revendication 1 dans lequel, ladite seconde amorce d'acides nucléiques est capable de lier une séquence selon SEQ ID NO. 8 dans une réaction d'amplification comprenant entre 1 mMol et 3 mMol de MgCl₂, à une température d'annelage comprise entre 45°C et 55°C.

3. Procédé selon la revendication 2 dans lequel, ladite seconde amorce d'acides nucléiques est choisie dans le groupe comprenant SEQ ID NO. 2, 3 et 4.

4. Procédé selon l'une quelconque des revendications précédentes dans lequel, ladite première amorce d'acides nucléiques consiste en un mélange d'amorces avec les séquences suivantes SEQ ID NO.1 et SEQ ID NO.5.

5. Procédé selon l'une quelconque des revendications précédentes dans lequel, la détection de l'étape (ii) est réalisée à l'aide d'une ou plusieurs sondes d'hybridation spécifiques d'une séquence dans une réaction d'hybridation dans laquelle, les sondes sont capables de lier le produit d'amplification produit dans l'étape (i) dans des conditions stringentes.

6. Procédé selon la revendication 5 dans lequel, l'hybridation est réalisée avec un matériau chromatographique qui comprend (i) une zone de réception de l'échantillon, (ii) une zone de séparation comprenant une région de liaison, dans laquelle un ou plusieurs acides nucléiques spécifiques d'une séquence sont immobilisés, (ii) et une zone qui absorbe un liquide en aval de la zone de séparation comprenant une région de liaison, (iii) et les sondes d'acide nucléique spécifique d'une séquence sont immobilisées via un lieur polymère et dans laquelle le procédé comprend les étapes suivantes consistant à :
(a) dénaturer, dans le cas d'acides nucléiques à double brin, l'acide nucléique à détecter et ensuite le neutraliser,
(b) appliquer l'acide nucléique à détecter à la zone de réception de l'échantillon dans un tampon circulant qui contient des agents de dénaturation faible,
(c) l'acide nucléique se déplaçant de la zone de réception de l'échantillon dans la direction de la zone d'absorption de liquide,
(d) mettre en contact l'acide nucléique à détecter dans la région de liaison du parcours de séparation avec la sonde d'acide nucléique spécifique d'une séquence et l'hybrider avec la sonde d'acide nucléique spécifique d'une séquence,
(e) détecter l'acide nucléique ou l'hybridation de l'acide nucléique avec la sonde d'acide nucléique spécifique d'une séquence via un marqueur qui est attaché à l'acide nucléique à détecter ou via la détection d'un marqueur du double brin de l'acide nucléique.

7. Kit pour réaliser une réaction selon l'une quelconque des revendications précédentes, comprenant une amorce d'acide nucléique selon SEQ ID NO.1 ou selon SEQ ID NO. 5 ou comprenant les deux.


### SEQUENCE LISTING

<110> Federal Republic of Germany represented by the Ministry of Health and Social Security, the latter represented by the President of the Robert Koch Institutes, Nordufer 20, 13353 Berlin, Germany
<120> Method for detecting methicillin resistant Staphylococcus aureus (MRSA)
<130> FBP13148
<160> 8
<170> Patent In version 3.1
<210> 1
   <211> 17
   <212> DNA
   <213> artificial
<400> 1
   ttatgataag cttctcc 17
<210> 2
   <211> 21
   <212> DNA
   <213> artificial
<400> 2
   aacgtttagg cccatacacc a 21
<210> 3
   <211> 21
   <212> DNA
   <213> artificial
<400> 3
   atgacattcc cacatcaaat g 21
<210> 4
   <211> 18
   <212> DNA
   <213> artificial
<400> 4
   acatcaaatg atgcgggt 18
<210> 5
   <211> 17
   <212> DNA
   <213> artificial
<400> 5
   ttatgatatg cttctcc 17
<210> 6
   <211> 46
   <212> DNA
   <213> artificial
<400> 6
   agtgtacgag gcattaagat tatgcgtgga gaagcatatc ataaat 46
<210> 7
   <211> 46
   <212> DNA
   <213> artificial
<400> 7
   agtgtacgag gcattaagat tatgcgagga gaagcttatc ataaaa 46
<210> 8
   <211> 370
   <212> DNA
   <213> artificial
<220>
   <221> misc_feature
   <222> (75)..(75)
   <223> a, c, g or t
<400> 8
   acgtttaggc ccatacacca agatagacat catagaagtt ccagacgaaa aagcaccaga      60
   aaatatgagt gacanagaaa ttgagcaagt aaaagaaaaa gaaggccaac gaatactagc     120
   caaaatcaaa ccacaatcca cagtcattac attagaaata caaggaaaga tgctatcttc     180
   cgaaggattg gcccaagaat tgaaccaacg catgacccaa gggcaaagcg actttgtttt     240
   cgtcattggc ggatcaaacg gcctgcacaa ggacgtctta caacgcagta actacgcact     300
   atcattcagc aaaatgacat tcccacatca aatgatgcgg gttgtgttaa ttgaacaagt     360
   gtacagagca                                                            370